(19) Europäisches Patentamt
European Patent Office
Office européen des brevets

(11) Veröffentlichungsnummer: **0 239 535**
**A2**

# (12) EUROPÄISCHE PATENTANMELDUNG

(21) Anmeldenummer: **87810146.8**

(22) Anmeldetag: **13.03.87**

(51) Int. Cl.⁴: **C 07 D 213/64**
A 01 N 47/42

(30) Priorität: **19.03.86 CH 1107/86 06.02.87 CH 442/87**

(43) Veröffentlichungstag der Anmeldung:
**30.09.87 Patentblatt 87/40**

(84) Benannte Vertragsstaaten:
**AT BE CH DE ES FR GB GR IT LI NL**

(71) Anmelder: **CIBA-GEIGY AG**
**Klybeckstrasse 141**
**CH-4002 Basel (CH)**

(72) Erfinder: **Böger, Manfred**
**Wilhelm Glockstrasse 14**
**D-7858 Weil am Rhein 5 (DE)**

**Drabek, Jozef, Dr.**
**Benkenstrasse 12**
**CH-4104 Oberwil (CH)**

## (54) N-Pyridyloxyphenyl-isothioharnstoffe, ihre Herstellung und Verwendung bei der Kontrolle von Schädlingen.

(57) Neue N-Pyridyloxyphenyl-isothioharnstoffe der Formel I

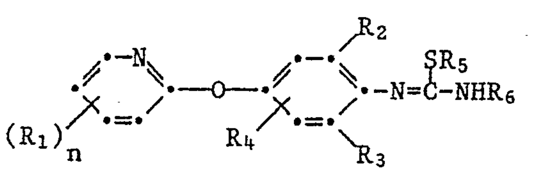

(I)

worin
$R_1$ je für Halogen, $C_1$-$C_3$-Alkyl, ein ein- oder mehrfach halogeniertes $C_1$-$C_3$-Alkyl oder $-COOR_7$,
$R_2$ $R_3$ und $R_7$ je für $C_1$-$C_5$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind,
$R_4$ für Wasserstoff oder Methyl,
$R_5$ für $C_1$-$C_6$-Alkyl,
$R_6$ für $C_1$-$C_{12}$-Alkyl, ein ein- oder mehrfach halogeniertes $C_1$-$C_{12}$-Alkyl, $C_1$-$C_6$-Alkoxy-$C_1$-$C_{12}$-alkyl, $C_3$-$C_8$-Cycloalkyl, ein ein-oder zweifach $C_1$-$C_3$-alkyliertes $C_3$-$C_8$-Cycloalkyl, $C_3$-$C_8$-Cycloalkyl-$C_1$-$C_4$-alkyl oder einen polycyclischen Alkylrest mit 7 bis 10 C-Atomen und
n für eine Zahl von 0 bis 4

stehen, und ihre Salze mit organischen oder anorganischen Säuren, Verfahren zu ihrer Herstellung, ihre Verwendung in der Schädlingsbekämpfung, sowie Schädlingsbekämpfungsmittel, welche mindestens eine Verbindung der Formel I oder ein aktives Zwischenprodukt enthalten, werden offenbart. Bevorzugtes Anwendungsgebiet ist die Bekämpfung von Schädlingen an Tieren und Pflanzen.

EP 0 239 535 A2

**Beschreibung**

N-Pyridyloxyphenyl-isothioharnstoffe, ihre Herstellung und Verwendung bei der Kontrolle von Schädlingen

Die vorliegende Erfindung betrifft N-Pyridyloxyphenyl-isothioharnstoffe, ihre Salze mit organischen oder anorganischen Säuren, Verfahren zu ihrer Herstellung, Schädlingsbekämpfungsmittel, die diese Verbindungen oder aktive Zwischenprodukte enthalten, sowie ihre Verwendung in der Schädlingsbekämpfung.

Die erfindungsgemässen Verbindungen entsprechen der Formel I

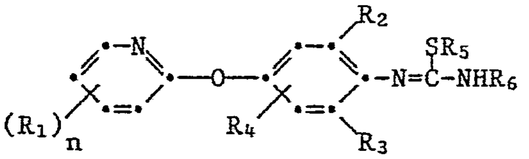

(I),

worin

$R_1$ je für Halogen, $C_1$-$C_3$-Alkyl, ein ein- oder mehrfach halogeniertes $C_1$-$C_3$-Alkyl oder $-COOR_7$,
$R_2$, $R_3$ und $R_7$ je für $C_1$-$C_5$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind,
R4 für Wasserstoff oder Methyl,
$R_5$ für $C_1$-$C_6$-Alkyl,
$R_6$ für C-$C_{12}$-Alkyl, ein ein- oder mehrfach halogeniertes $C_1$-$C_{12}$-Alkyl, $C_1$-$C_6$-Alkoxy-$C_1$-$C_{12}$-alkyl, $C_3$-$C_8$-Cycloalkyl, ein ein- oder zweifach $C_1$-$C_3$-alkyliertes $C_3$-$C_8$-Cycloalkyl, $C_3$-$C_8$-Cycloalkyl-$C_1$-$C_4$-alkyl oder einen polycyclischen Alkylrest mit 7 bis 10 C-Atomen und
n für eine Zahl von 0 bis 4
stehen.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle können geradkettig oder verzweigt sein. Als Beisiele solcher Alkyle seien Methyl, Aethyl, Propyl, Isopropyl, Butyl, i-Butyl, sek.Butyl, tert. Butyl oder Pentyl, Hexyl, Heptyl, Octyl und ihre Isomeren genannt.

Die als Substituenten in Betracht kommenden ein- oder mehrfach halogenierten $C_1$-$C_{12}$-Alkyle können geradkettig oder verzweigt und nur teilweise oder auch perhalogeniert sein, wobei für die Halogene die oben gegebene Definition gilt. Besonders geeignete Beispiele solcher Substituenten sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$; das ein-bis neunfach durch Fluor, Chlor und/oder Brom substituierte Butyl oder eines seiner Isomeren wie z.B. $CF(CF_3)CHFCF_3$ oder $CH_2(CF_2)_2CF_3$.

Die als Substituenten in Betracht kommenden Alkoxyalkyle können geradkettig oder verzweigt sein. Beispiele solcher Gruppen sind u.a. Methoxymethyl, Methoxyäthyl, Aethoxyäthyl, Methoxypropyl, Aethoxypropyl, Propoxypropyl, Methoxybutyl, Aethoxybutyl, Propoxybutyl oder Butoxybutyl.

Bei den als Substituenten in Betracht kommenden Cycloalkylen handelt es sich beispielsweise um Cyclopropyl, Cyclobutyl, Cyclopentyl oder Cyclohexyl. Diese können ein- oder zweifach mit einem $C_1$-$C_3$-Alkylrest substituiert und/oder über eine $C_1$-$C_4$-Alkylenbrücke mit dem Rest des Moleküls verbunden sein.

Die als Substituenten in Betracht kommenden polycyclischen Alkylreste können 7 bis 10 C-Atome aufweisen. Beispiele solcher Reste sind u.a. der Bicyclo[2,1,0]heptyl-, der Bicyclo[3,2,1]octyl- oder der Bicyclo[3,3,1]nonyl-Rest.

Die Verbindungen der Formel I können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Salpetersäure, verschiedene Phosphorsäuren, Schwefelsäure, Essigsäure, Propionsäure, Buttersäure, Valeriansäure, Oxalsäure, Malonsäure, Maleinsäure, Fumarsäure, Milchsäure, Weinsäure oder Salicylsäure.

Der Pyridylrest kann mehrfach, je nach der Grösse von n, durch $R_1$ substituiert sein. Ist n grösser als 1, so können die verschiedenen $R_1$ gleiche oder auch verschiedene Bedeutung haben.

Unter den Verbindungen der Formel I stehen diejenigen im Vordergrund, in denen
$R_1$ je für Fluor, Chlor oder ein ein- oder mehrfach halogeniertes $C_1$-$C_3$-Alkyl,
$R_2$ und $R_3$ je für $C_1$-$C_3$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind,
$R_4$ für Wasserstoff,
$R_5$ für $C_1$-$C_3$-Alkyl,

$R_6$ für $C_1$-$C_8$-Alkyl, $C_1$-$C_4$-Alkoxy-$C_1$-$C_4$-alkyl, $C_3$-$C_6$-Cycloalkyl, ein- oder zweifach $C_1$-$C_3$-alkyliertes $C_3$-$C_6$-Cycloalkyl, $C_3$-$C_6$-Cycloalkyl-$C_1$-$C_3$-alkyl und
n für die Zahl 1 oder 2
stehen.

Dabei sind diejenigen Verbindungen der Formel I bevorzugt, in denen
$R_1$ je für Chlor oder $CF_2CFCl_2$,
$R_2$ und $R_3$ je für Methyl oder Aethyl, mit der Massgabe, dass $R_2$ und $R_3$ nicht gleichzeitig Methyl bedeuten, wenn $R_6$ tert.Butyl und $R_1$ Chlor und/oder Trilfuormethyl sind,
$R_4$ für Wasserstoff,
$R_5$ für Methyl,
$R_6$ für $C_1$-$C_5$-Alkyl oder $C_3$-$C_6$-Cycloalkyl und
n für 2
stehen.

Beispiele von Verbindungen der Formel I sind u.a.

| $R_1$ | $R_2$ | $R_3$ | $R_4$ | $R_5$ | $R_6$ |
|---|---|---|---|---|---|
| 3,5-Cl | $CH_3$ | $CH_3$ | H | $CH_3$ | s-$C_4H_9$ |
| 3,5-Cl | n-$C_3H_7$ | n-$C_3H_7$ | H | $CH_3$ | t-$C_4H_9$ |
| 3,5-Cl | $C_2H_5$ | s-$C_4H_9$ | H | $CH_3$ | t-$C_4H_9$ |
| 3,5-Cl | $CH_3$ | i-$C_5H_{11}$ | H | $CH_3$ | t-$C_4H_9$ |
| 3,5-Cl | $CH_3$ | $CH_3$ | H | $CH_3$ | CH(i-$C_3H_7$)$_2$ |
| 3,5-Cl | $CH_3$ | $CH_3$ | H | $CH_3$ | $CH(C_2H_5)_2$ |
| 3,5-Cl | $C_2H_5$ | $C_2H_5$ | H | $C_2H_5$ | t-$C_4H_9$ |
| 3,5-Cl | $C_2H_5$ | $CH_3$ | H | n-$C_6H_{13}$ | t-$C_4H_9$ |
| 3,5-Cl | $CH_3$ | $CH_3$ | $CH_3$ | $CH_3$ | s-$C_5H_{11}$ |
| 3,5-Cl | $CH_3$ | $CH_3$ | $C_2H_5$ | $CH_3$ | s-$C_4H_9$ |
| 3,5-Cl | $CH_3$ | $C_2H_5$ | $CH_3$ | $CH_3$ | i-$C_4H_9$ |
| 4-$COOCH_3$, 6-Cl | $CH_3$ | $CH_3$ | H | $CH_3$ | t-$C_4H_9$ |
| 4-$COOC_5H_{11}$(n), 6-Cl | $CH_3$ | $CH_3$ | H | $CH_3$ | t-$C_4H_9$ |
| 5-n-$C_3H_7$ | $CH_3$ | $CH_3$ | H | $CH_3$ | t-$C_4H_9$ |

Die erfindungsgemässen Verbindungen der Formel I können nach im Prinzip bekannten Methoden hergestellt werden, indem man z.B. einen Thioharnstoff der Formel II

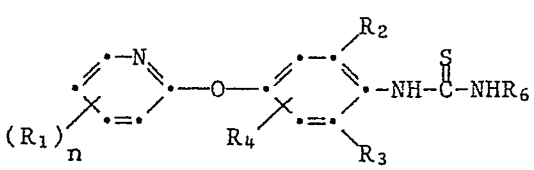

(II)

mit einem Alkylierungsmittel der Formel III
$R_5$-X (III)
in Gegenwart einer Base umsetzt, wobei in den Formeln II und III $R_1$, $R_2$, $R_3$, $R_4$, $R_5$, $R_6$ und n die für Formel I angegebenen Bedeutungen haben und X für eine Abgangsgruppe wie z.B. ein Halogenatom, insbesondere Chlor, Brom oder Jod, stehen.

Das Verfahren wird zweckmässigerweise bei einer Temperatur zwischen 0 und 100°C, bei normalem oder leicht erhöhtem Druck und vorzugsweise in Gegenwart eines gegenüber den Reaktionsteilnehmern inerten Lösungs- oder Verdünnungsmittels durchgeführt. Als Lösungs-oder Verdünnungsmittel eignen sich z.B.

Aether oder ätherartige Verbindungen wie z.B. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; oder Alkohole wie Methanol oder Aethanol.

Geeignete Basen können organischen oder anorganischen Ursprungs sein wie z.B. Natriumhydrid, Natrium- oder Calciumcarbonat, tertiäre Amine wie Triäthylamin, Triäthylendiamin oder 4-Dimethylaminopyridin oder Pyridin.

Die Thioharnstoffe der Formel II ihrerseits können ebenfalls nach im Prinzip bekannten Verfahren hergestellt werden, z.B. indem man ein Isothiocyanat der Formel IV

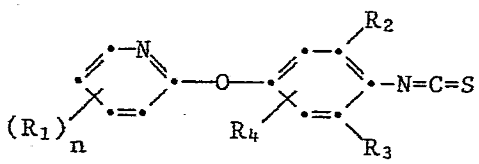

(IV)

mit einem Amin der Formel V

$H_2N$-$R_6$ (V)

umsetzt, wobei in den Formeln IV und V $R_1$, $R_2$, $R_3$, $R_4$, $R_6$ und n die für Formel I angegebenen Bedeutungen haben.

Das Verfahren zur Herstellung der Verbindungen der Formel II wird zweckmässigerweise in Gegenwart eines gegenüber den Reaktionsteilnehmern inerten Lösungs- oder Verdünnungsmittels, bei einer Reaktionstemperatur von 0 bis 100°C unter normalem Druck durchgeführt. Geeignete Lösungs- und Verdünnungsmittel sind beispielsweise schon für das Verfahren zur Herstellung der Verbindungen der Formel I aufgezählt worden. Dabei ist zu beachten, dass sich Alkohole hier nicht als Lösungsmittel eignen.

Die Isothiocyanate der Formel IV ihrerseits können nach im Prinzip bekannten Methoden hergestellt werden, z.B. indem man ein Pyridyloxyanilin der Formel VI

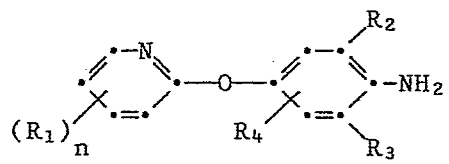

(VI)

mit Thiophosgen umsetzt, wobei $R_1$, $R_2$, $R_3$, $R_4$ und n die für Formel I angegebenen Bedeutungen haben.

Das Verfahren zur Herstellung der Verbindungen der Formel IV wird zweckmässigerweise in Gegenwart einer organischen oder anorganischen Base, wie z.B. Triäthylamin oder Calciumcarbonat und einem gegenüber den Reaktionsteilnehmern inerten Lösungs- oder Verdünnungsmittels, bei einer Temeratur zwischen 0 und 100°C und bei normalem Druck durchgeführt. Geeignete Lösungs- und Verdünnungsmittel sind u.a. die für das Verfahren zur Herstellung der Verbindungen der Formel I aufgezählten oder Dichlormethan. Dabei ist zu beachten, dass sich Alkohle hier nicht als Lösungsmittel eignen.

Die Pyridyloxyaniline der Formel VI ihrerseits können nach im Prinzip bekannten Methoden hergestellt werden, z.B. indem man ein Hydroxyanilin der Formel VII

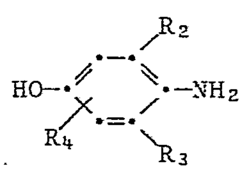

(VII)

mit einem Halogenpyridin der Formel VIII

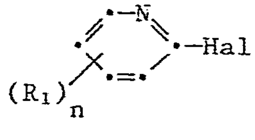

(VIII)

umsetzt, wobei $R_1$, $R_2$, $R_3$, $R_4$ und n die für Formel I angegebenen Bedeutungen haben und Hal für ein Halogenatom, insbesondere Fluor, Chlor oder Brom, steht.

Das Verfahren zur Herstellung der Verbindungen der Formel VI wird zweckmässigerweise in Gegenwart einer anorganischen oder organischen Base wie z.B. einem Alkalihydroxid oder -carbonat, und einem gegenüber den Reaktionsteilnehmern inerten, vorzugsweise polaren Lösungs- oder Verdünnungsmittels, bei

einer Temperatur zwischen 0 und 100°C und bei normalem Druck durchgeführt. Geeignete Lösungs-und Verdünnungsmittel wurden schon für das Verfahren zur Herstellung der Verbindungen der Formel I aufgezählt; besonders geeignet ist auch Dimethylsulfoxid.

Die Verbindungen der Formeln II, III, IV, V VI, VII und VIII sind bekannt oder können nach im Prinzip bekannten Methoden hergestellt werden.

Ueberraschenderweise wurde gefunden, dass die erfindungsgemässen Verbindungen der Formel I, sowie die Zwischenprodukte der Formel II bei günstiger Warmblüter- und Pflanzenverträglichkeit wertvolle Wirkstoffe in der Schädlingsbekämpfung sind. So eignen sich die Verbindungen der Formeln I und II z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formeln I und II von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus-und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formeln I und II entspricht einer Abtötungsrate (Mortalität) von mindestens 50-60 % der erwähnten Schädlinge.

Die Wirkung der Verbindungen der Formeln I und II bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formeln I und II werden in unveränderter Form oder vorzugsweise zusammen mit in der Formulierungstechnik üblichen, inerten, pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formeln I und/oder II, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder II oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate,

Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder II oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

1.1. Zwischenprodukte

1.1.1. Pyridyloxyaniline

1.1.1.1. 2-Aethyl-6-methyl-4-(3',5'-dichlorpyrid-2'-yloxy)-anilin

23,1 g 2-Aethyl-4-hydroxy-6-methyl-anilin und 35,7 g pulverisiertes Kaliumcarbonat werden zu 90 ml Dimethylsulfoxid gegeben und eine Stunde lang bei Raumtemperatur gerührt. Unter ständigem Rühren wird nun eine Lösung von 27,4 g 2,3,5-Trichlorpyridin in 30 ml Dimethylsulfoxid tropfenweise zugegeben, worauf die Temperatur auf 80-90°C erhöht und das Reaktionsgemisch 4 Stunden lang nachgerührt wird. Anschliessend wird das Reaktionsgemisch am Hochvakuum eingeengt und der Rückstand in je 100 ml Dichlormethan und Wasser aufgenommen. Die organische Phase wird mit Wasser gewaschen und über Natriumsulfat getrocknet, worauf das Lösungsmittel abdestilliert wird. Der Rückstand wird aus Hexan umkristallisiert. Die Titelverbindung der Formel

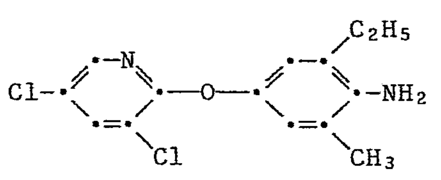

(Verb. 1.1.1.1.)

liegt nun in Form hell-beiger Kristalle vor; Smp. 104-106°C.

Auf analoge Weise werden die folgenden Verbindungen hergestellt:

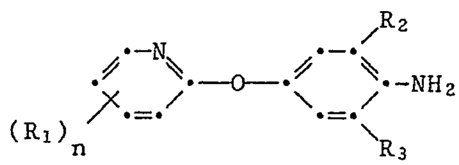

| Verb. Nr. | $R_1$ | $R_2$ | $R_3$ | n | Smp. °C |
|---|---|---|---|---|---|
| 1.1.1.2. | 3-Cl, 5-Cl | $CH_3$ | $CH_3$ | 2 | 118-120 |
| 1.1.1.3. | 3-F, 5-Cl | $CH_3$ | $CH_3$ | 2 | 125-128 |
| 1.1.1.4. | 3-Cl, 5-Cl | $C_2H_5$ | $C_2H_5$ | 2 | 105-106 |
| 1.1.1.5. | 3-Cl, 5-$CF_2CFCl_2$ | $CH_3$ | $CH_3$ | 2 | 139-141 |
| 1.1.1.6. | 3-Cl, 5-$CF_2CFCl_2$ | $CH_3$ | $C_2H_5$ | 2 | 104-107 |
| 1.1.1.7. | 4-Cl, 6-Cl | $CH_3$ | $CH_3$ | 2 | 119-121 |

1.1.2. Pyridyloxyphenyl-isothiocyanate

1.1.2.1. 2-Aethyl-6-methyl-4-(3',5'-dichlorpyrid-2'-yloxy)-phenyl-isothiocyanat

7,5 g Thiophosgen, 10 g Calciumcarbonat und 80 ml Dichlormethan werden mit 40 ml Wasser verrührt. Zu diesem Gemisch wird bei 0-5°C tropfenweise eine Lösung von 14,9 g 2-Aethyl-6-methyl-4-(3',5'-dichlorpyrid-2'-yloxy)-anilin in 30 ml Dichloräthan gegeben. Das Reaktionsgemisch wird 3 Stunden lang bei Raumtemperatur gerührt und dann filtriert. Aus dem Filtrat wird die organische Phase abgetrennt; diese wird zweimal mit 30 ml Wasser gewaschen und über Natriumsulfat getrocknet und schliesslich eingedampft. Die Titelverbindung der Formel

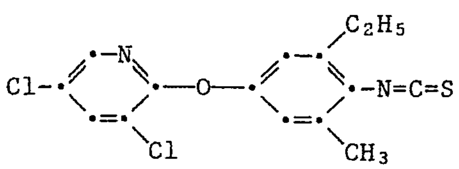

(Verb. 1.1.2.1.)

liegt als gelbes Oel vor, das beim Stehenlassen kristallisiert; Smp. 48-50°C.

Auf analoge Weise werden die folgenden Verbindungen hergestellt:

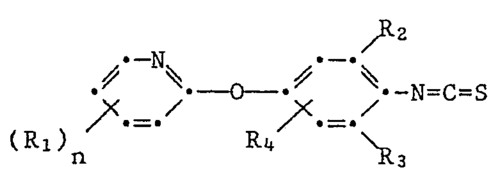

| Verb. Nr. | $R_1$ | $R_2$ | $R_3$ | $R_4$ | n | Phys. Daten |
|---|---|---|---|---|---|---|
| 1.1.2.2. | 3-Cl, 5-Cl | $CH_3$ | $CH_3$ | H | 2 | Smp. 89-91°C |
| 1.1.2.3. | 3-F, 5-Cl | $CH_3$ | $CH_3$ | H | 2 | Sdp. 168°C/3 mbar |
| 1.1.2.4. | 3-Cl, 5-Cl | $C_2H_5$ | $C_2H_5$ | H | 2 | Sdp. 175°C/2 mbar |
| 1.1.2.5. | 3-Cl, 5-$CF_2CFCl_2$ | $CH_3$ | $CH_3$ | H | 2 | Smp. 79-81°C |
| 1.1.2.6. | 3-Cl, 5-$CF_2CFCl_2$ | $CH_3$ | $C_2H_5$ | H | 2 | Sdp. 170°C/3 mbar |
| 1.1.2.7. | 4-Cl, 6-Cl | $CH_3$ | $CH_3$ | H | 2 | Wachs |

1.1.3. Pyridyloxyphenyl-thioharnstoffe

1.1.3.1. N-[2-Aethyl-6-methyl-4-(3′,5′-dichlorpyrid-2′-yloxy)-phenyl]-N′-(tert.butyl)-thioharnstoff

13,1 g 2-Aethyl-6-methyl-4-(3′,5′-dichlorpyrid-2′-yloxy)-phenylisothiocyanat und 3,7 g tert.Butylamin werden in 30 ml Toluol aufgenommen und 3 Stunden lang bei 45°C gerührt. Nach dem Abkühlen wird die Reaktionslösung mit 50 ml Toluol verdünnt, mit Wasser gewaschen und über Natriumsulfat getrocknet. Die trockene Lösung wird eingedampft und der Rückstand mit Hexan verrührt. Die Titelverbindung der Formel

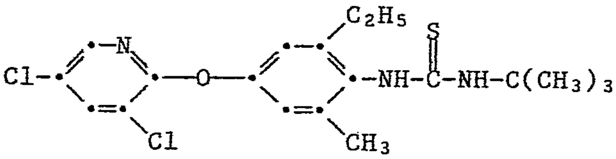 (Verb. 1.1.3.1.)

liegt als hell-beiges Pulver vor; Smp. 96-98°C. Auf analoge Weise werden die folgenden Verbindungen hergestellt:

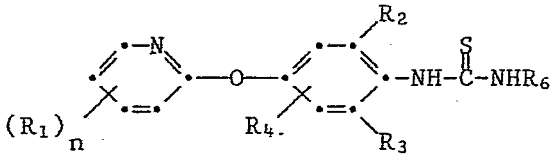

| Verb. Nr. | $R_1$ | $R_2$ | $R_3$ | $R_4$ | $R_6$ | n | Smp. °C |
|---|---|---|---|---|---|---|---|
| 1.1.3.2. | 3-Cl, 5-Cl | $CH_3$ | $CH_3$ | H | $-(CH_2)_3O(CH_2)_3CH_3$ | 2 | 68-70 |
| 1.1.3.3. | 3-Cl, 5-Cl | $CH_3$ | $CH_3$ | H | 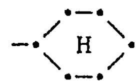 | 2 | 146-149 |
| 1.1.3.4. | 3-Cl, 5-Cl | $CH_3$ | $CH_3$ | H | $-C(CH_3)_2-CH_2-C(CH_3)_3$ | 2 | 138-140 |
| 1.1.3.5. | 3-Cl, 5-Cl | $CH_3$ | $CH_3$ | H | $-CH(CH_3)-$ 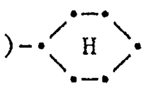 | 2 | 143-145 |
| 1.1.3.6. | 3-Cl, 5-Cl | $CH_3$ | $CH_3$ | H | $-CH_2-CH(CH_3)_2$ | 2 | 104-106 |
| 1.1.3.7. | 3-Cl, 5-Cl | $CH_3$ | $CH_3$ | H | $-C(CH_3)_2-C_2H_5$ | 2 | 118-121 |
| 1.1.3.8. | 3-Cl, 5-Cl | $CH_3$ | $CH_3$ | H | -CH 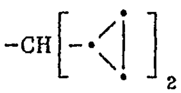 | 2 | 125-127 |
| 1.1.3.9. | 3-Cl, 5-Cl | $CH_3$ | $CH_3$ | H | $-CH_2-$ 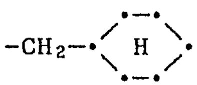 | 2 | 131-133 |
| 1.1.3.10. | 3-Cl, 5-Cl | $CH_3$ | $CH_3$ | H | 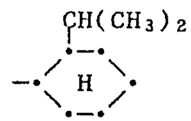 | 2 | 178-180 |
| 1.1.3.11. | 3-Cl, 5-Cl | $CH_3$ | $CH_3$ | H | 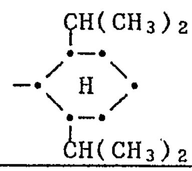 | 2 | 174-176 |

| Verb. Nr. | $R_1$ | $R_2$ | $R_3$ | $R_4$ | $R_6$ | n | Smp. °C |
|---|---|---|---|---|---|---|---|
| 1.1.3.12. | 3-Cl, 5-Cl | $CH_3$ | $CH_3$ | H | 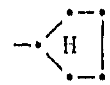 | 2 | amorph |
| 1.1.3.13. | 3-Cl, 5-Cl | $CH_3$ | $CH_3$ | H | $-CH(CH_3)-$ 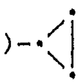 | 2 | 55 |
| 1.1.3.14. | 3-Cl, 5-Cl | $CH_3$ | $CH_3$ | H | $-C(CH_3)_2-$ 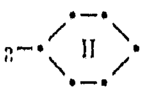 | 2 | 149-151 |
| 1.1.3.15. | 3-Cl, 5-Cl | $CH_3$ | $CH_3$ | H | $-CH(CH_3)_2$ | 2 | 164-166 |
| 1.1.3.16. | 3-Cl, 5-Cl | $C_2H_5$ | $C_2H_5$ | H | $-C(CH_3)_3$ | 2 | 168-169 |
| 1.1.3.17. | 3-F, 5-Cl | $CH_3$ | $CH_3$ | H | $-CH(CH_3)_2$ | 2 | 113-115 |
| 1.1.3.18. | 3-Cl, 5-$CF_2CFCl_2$ | $CH_3$ | $CH_3$ | H | $-C(CH_3)_3$ | 2 | 138-140 |
| 1.1.3.19. | 3-Cl, 5-$CF_2CFCl_2$ | $CH_3$ | $CH_3$ | H | $-CH(CH_3)_2$ | 2 | 160-162 |
| 1.1.3.20. | 3-Cl, 5-$CF_2CFCl_2$ | $CH_3$ | $CH_3$ | H | 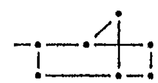 | 2 | 151-153 |
| 1.1.3.21. | 3-Cl, 5-$CF_2CFCl_2$ | $CH_3$ | $CH_3$ | H | $-C(CH_3)_2-C_2H_5$ | 2 | 139-141 |
| 1.1.3.22. | 3-Cl, 5-$CF_2CFCl_2$ | $CH_3$ | $C_2H_5$ | H | $-C(CH_3)_3$ | 2 | amorph |
| 1.1.3.23. | 4-Cl, 6-Cl | $CH_3$ | $CH_3$ | H | $-CH(CH_3)_2$ | 2 | 194-196 |

| Verb. Nr. | $R_1$ | $R_2$ | $R_3$ | $R_4$ | $R_6$ | n | Smp. °C |
|---|---|---|---|---|---|---|---|
| 1.1.3.24 | 4-Cl, 6-Cl | $CH_3$ | $CH_3$ | H | $-CH(CH(CH_3)_2)_2$ | 2 | 181-183 |
| 1.1.3.25. | 3-Cl, 5-Cl | $CH_3$ | $CH_3$ | H | $-CH(CH_3)-C_2H_5$ | 2 | 138-140 |
| 1.1.3.26. | 3-Cl, 5-Cl | $CH(CH_3)_2$ | $CH(CH_3)_2$ | H | $-C(CH_3)_3$ | 2 | 158-160 |
| 1.1.3.27. | 3-Cl, 5-Cl | $CH(CH_3)_2$ | $CH(CH_3)_2$ | H | $-CH(CH_3)_2$ | 2 | 159-161 |
| 1.1.3.28. | 3-Cl, 5-Cl | $CH(CH_3)_2$ | $CH(CH_3)_2$ | H | $-CH(CH(CH_3)_2)_2$ | 2 | 163-166 |
| 1.1.3.29. | 3-Cl, 5-$CF_3$ | $CH(CH_3)_2$ | $CH(CH_3)_2$ | H | $-C(CH_3)_3$ | 2 | 170-172 |
| 1.1.3.30. | 3-Cl, 5-$CH_3$ | $CH_3$ | $CH_3$ | H | $-CH(CH_3)_2$ | 2 | 130-131 |
| 1.1.3.31. | 3-Cl, 5-Cl | $CH_3$ | $C_2H_5$ | H | $-CH(CH_3)_2$ | 2 | amorph |
| 1.1.3.32. | 3-Cl, 5-Cl | $C_2H_5$ | $C_2H_5$ | H | $-CH(CH_3)_2$ | 2 | ca. 60 |

1.2. Endprodukte

1.2.1. N-[2-Aethyl-6-methyl-4-(3',5'-dichlorpyrid-2'-yloxy)-phenyl]-N'-(tert.butyl)-S-methyl-isothioharnstoff

3,85 g N-[2-Aethyl-6-methyl-4-(3',5'-dichlorpyrid-2'-yloxy)-phenyl]-N'-(tert.butyl)-thioharnstoff werden in 40 ml Aethanol gegeben, mit 1,7 g Methyljodid versetzt und während 3 Stunden unter leichtem Rückfluss gerührt. Anschliessend wird die Reaktionslösung eingedampft und der Rückstand in Dichlormethan und 10%iger Natriumcarbonat-Lösung aufgenommen. Die organische Phase wird nun mit Wasser gewaschen, über Natriumsulfat getrocknet und zur Trockne eingedampft. Die Titelverbindung der Formel

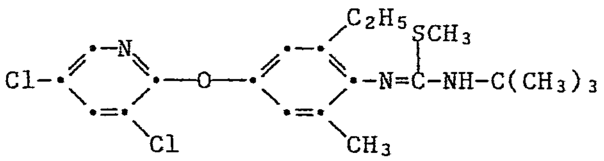 (Verb. 1.2.1.)

liegt als hellgelbes Oel vor; Brechungsindex $n_D^{49}$ : 1,5610.

Auf analoge Weise werden die folgenden Verbindungen hergestellt:

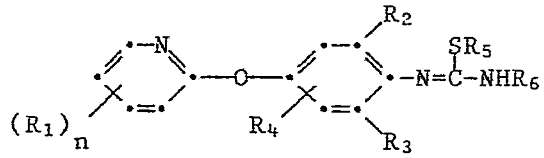

| Verb. Nr. | $R_1$ | $R_2$ | $R_3$ | $R_4$ | $R_5$ | $R_6$ | n | phys. Daten |
|---|---|---|---|---|---|---|---|---|
| 1.2.2 | 3-Cl, 5-Cl | $CH_3$ | $CH_3$ | H | $CH_3$ | $-CH(CH_3)-$ 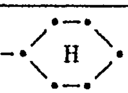 | 2 | $n_D^{49}$ 1,5840 |
| 1.2.3 | 3-Cl, 5-Cl | $CH_3$ | $CH_3$ | H | $CH_3$ | $-CH_2-CH(CH_3)_2$ | 2 | |
| 1.2.4 | 3-Cl, 5-Cl | $CH_3$ | $CH_3$ | H | $CH_3$ | $-C(CH_3)_2-C_2H_5$ | 2 | $n_D^{49}$ 1,5820 |
| 1.2.5 | 3-Cl, 5-Cl | $CH_3$ | $CH_3$ | H | $CH_3$ | -CH 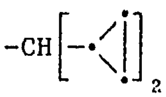 | 2 | Smp. 88-90°C |
| 1.2.6 | 3-Cl, 5-Cl | $CH_3$ | $CH_3$ | H | $CH_3$ | 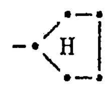 | 2 | $n_D^{49}$ 1,5980 |
| 1.2.7 | 3-Cl, 5-Cl | $CH_3$ | $CH_3$ | H | $CH_3$ | $-CH(CH_3)-$ 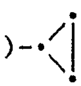 | 2 | $n_D^{49}$ 1,5900 |
| 1.2.8 | 3-Cl, 5-Cl | $C_2H_5$ | $C_2H_5$ | H | $CH_3$ | $-C(CH_3)_3$ | 2 | $n_D^{49}$ 1,5774 |
| 1.2.9 | 3-Cl, 5-$CF_2CFCl_2$ | $CH_3$ | $CH_3$ | H | $CH_3$ | $-C(CH_3)_3$ | 2 | $n_D^{49}$ 1,5505 |
| 1.2.10 | 3-Cl, 5-$CF_2CFCl_2$ | $CH_3$ | $CH_3$ | H | $CH_3$ | $-CH(CH_3)_2$ | 2 | $n_D^{49}$ 1,5560 |
| 1.2.11 | 3-Cl, 5-Cl | $CH(CH_3)_2$ | $CH(CH_3)_2$ | H | $CH_3$ | $-C(CH_3)_3$ | 2 | amorph |
| 1.2.12 | 3-Cl, 5-Cl | $CH(CH_3)_2$ | $CH(CH_3)_2$ | H | $CH_3$ | $-CH(CH(CH_3)_2)_2$ | 2 | Smp. 109-110°C |

| Verb. Nr. | $R_1$ | $R_2$ | $R_3$ | $R_4$ | $R_5$ | $R_6$ | n | phys. Daten |
|---|---|---|---|---|---|---|---|---|
| 1.2.13 | 3-Cl, 5-$CF_3$ | $CH(CH_3)_2$ | $CH(CH_3)_2$ | H | n-$C_4H_9$ | -$C(CH_3)_3$ | 2 | $n_D^{40}$ 1,5239 |
| 1.2.14 | 3-Cl, 5-$CH_3$ | $CH_3$ | $CH_3$ | H | $CH_3$ | -$CH(CH_3)_2$ | 2 | Smp. 111-113°C |
| 1.2.15 | 3-Cl, 5-Cl | $CH_3$ | $C_2H_5$ | H | $CH_3$ | -$CH(CH_3)_2$ | 2 | $n_D^{24}$ 1,5959 |
| 1.2.16 | 3-Cl, 5-Cl | $C_2H_5$ | $C_2H_5$ | H | $CH_3$ | -$CH(CH_3)_2$ | 2 | $n_D^{24}$ 1,5880 |
| 1.2.17 | 3-Cl, 5-Cl | $CH_3$ | $CH_3$ | H | $CH_3$ | -$CH(CH_3)_2$ | 2 | Harz |
| 1.2.18 | 3-Cl, 5-Cl | $CH_3$ | $CH_3$ | H | n-$C_6H_{13}$ | -$CH(CH_3)_2$ | 2 | Harz |
| 1.2.19 | 3-Cl, 5-Cl | $CH_3$ | $CH_3$ | H | $CH_3$ | -$CH_2CF_3$ | 2 | 102-103°C |

1.2.20.N-[2,6-Dimetyhl-4-(3',5'-dichlorpyrid-2'-yloxy)-phenyl]-N'-isopropyl-S-methyl-isothioharnstoff 4-Toluolsulfonsäuresalz

5,0 g N-[2,6-Dimethyl-4-(3',5'-dichlorpyrid-2'-yloxy)-phenyl]-N'-isopropyl-S-methyl-isothioharnstoff werden in 20 ml absolutem Diäthyläther gelöst und tropfenweise mit 2,2 g 4-Toluolsulfonsäure, gelöst in 10 ml Diäthyläther versetzt. Die Reaktionslösung wird 3 Stunden lang bei Raumtemperatur nachgerührt. Der entstandene Niederschlag wird abgenutscht und getrocknet. Die Titelverbindung der Formel

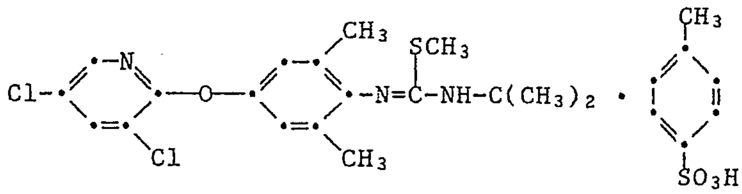

(Verb. 1.2.20)

liegt als weisses, kristallines Pulver vor. Smp. 198-200° C.

Auf analoge Weise werden die folgenden Verbindungen hergestellt:

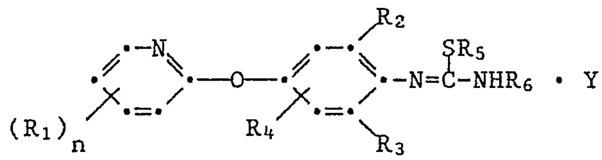

| Verb. Nr. | $R_1$ | $R_2$ | $R_3$ | $R_4$ | $R_5$ | $R_6$ | n | Y | Smp. C° |
|---|---|---|---|---|---|---|---|---|---|
| 1.2.21 | 3-Cl, 5-Cl | $CH_3$ | $CH_3$ | H | $CH_3$ | $-CH(CH_3)_2$ | 2 | $(COOH)_2$ | 175 Zers. |
| 1.2.22 | 3-Cl, 5-Cl | $CH_3$ | $CH_3$ | H | $CH_3$ | $-CH(CH_3)_2$ | 2 | HJ | > 250 |

Die HJ-Salze können auch direkt im Verfahren 1.2.1. gewonnen werden, wenn nach der Zugabe von Methyljodid der Niederschlag isoliert und wie oben gereinigt wird.

Beispiel 2: Formulierungen von Wirkstoffen der Formeln I und II gemäss den Herstellungsbeispielen 1.1.3 und 1.2.

(% = Gewichtsprozent)

| 2.1 Emulsions-Konzentrate | a) | b) |
|---|---|---|
| Wirkstoff gemäss den Herstellungs-beispielen 1.1.3 und 1.2 | 10 % | 25 % |
| Ca-Dodecylbenzolsulfonat | - | 5 % |
| Ricinusöl-polyäthylenglykoläther (36 Mol AeO) | 25 % | 5 % |
| Tributylphenol-polyäthylenglykoläther (30 Mol AeO) | - | - |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss den Herstellungsbeispielen 1.1.3 und 1.2 | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3 Granulate | a) | b) |
|---|---|---|
| Wirkstoff gemäss den Herstellungsbeispielen 1.1.3 und 1.2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

2.4 Extruder Granulat
Wirkstoff gemäss den Herstellungsbeispielen 1.1.3 und 1.2 10%

Na-Ligninsulfonat 2%
Carboxymethylcellulose 1%
Kaolin 87%

Der Wirkstoff wird mit den Zusatzstoffen vermischt, vermahlen und mit Wasser angefeuchtet. Dieses Gemisch wird extrudiert und anschliessend im Luftstrom getrocknet.

2.5 Umhüllungs-Granulat

Wirkstoff gemäss den Herstellungsbeispielen 1.1.3 und 1.2 3%
Polyäthylenglykol (MG 200) 3%
Kaolin 94%

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.6 Stäubemittel | a) | b) | c) | d) |
|---|---|---|---|---|
| Wirkstoff gemäss den Herstellungsbeispielen 1.1.3 und 1.2 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7 Spritzpulver | a) | b) | c) |
|---|---|---|---|
| Wirkstoff gemäss den Herstellungsbeispielen 1.1.3 und 1.2 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8 Suspensions-Konzentrat

Wirkstoff gemäss den Herstellungsbeispielen 1.1.3 und 1.2 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff wird mit den Zusatzstoffen innig vermischt. Man erhält so ein

Suspensions-Konzentrat, aus welchem durch Verdünnen mit Wasser Suspensionen jeder gewünschten Konzentration hergestellt werden können.

Beispiel 3: Biologische Prüfungen

3.1 Wirkung gegen Musca domestica

50 g frisch zubereitetes CSMA-Nährsubstrat für Maden werden in einen Becher eingewogen. Von einer 1 gew.%igen acetonischen Lösung des Wirkstoffes werden 5 ml auf das im Becher befindliche Nährsubstrat pipettiert. Nach dem Durchmischen des Substrates lässt man das Aceton mindestens 20 Stunden lang verdampfen.

Dann werden 25 eintägige Maden von Musca domestica in den das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in einem mit einem Siebdeckel verschlossenen Gefäss deponiert.

Die ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen gemäss den Beispielen 1.1.3. und 1.2. zeigen gute Wirkung im obigen Test.

3.2 Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss den Beispielen 1.1.3. und 1.2. zeigen in diesem Test gute Wirkung gegen Lucilia sericata.

3.3 Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss den Beispielen 1.1.3. und 1.2. zeigen gute Wirkung in diesem Test.

3.4 Insektizide Frassgift-Wirkung

Baumwollpflanzen (ca. 20 cm hoch) werden mit einer wässrigen Wirkstoffemulsion (erhalten aus einem 10%igen emulgierbaren Konzentrat) besprüht, wobei die Wirkstoffemulsion 100 ppm der zu prüfenden Verbindung enthält.

Nach dem Antrocknen des Belages werden die Baumwollpflanzen je mit Spodoptera littoralis- und Heliothis virescens-Larven im dritten larvalen Stadium besetzt. Der Versuch wird bei 24°C und 60 % relativer Luftfeuchtigkeit durchgeführt. In Abständen von jeweils 24 Stunden werden Mortalität sowie Entwicklungs- und Häutungsstörungen der angesetzten Larven bestimmt.

Verbindungen gemäss den Beispielen 1.1.3. und 1.2. zeigen gute Wirkung in diesem Test.

3.5 Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodoptera littoralis oder Heliothis virescens (dazu werden je 2 Blätter einer Pflanze in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven,

b) larvale Entwicklungs- und Häutungshemmung,

c) Frassschaden (Schabfrass und Lochfrass),

d) Schlupfrate (Anzahl der aus den Eiern geschlüpften Larven).

Verbindungen gemäss den Beispielen 1.1.3. und 1.2. zeigen in einer Konzentration von 400 ppm gute Gesamt-Wirksamkeit in obigem Test.

3.6 Ovizide Wirkung auf Spodoptera littoralis

Auf Filterpapier abgelegte Eier von Spodoptera littoralis werden aus dem Papier ausgeschnitten und in eine 0,05gew.%ige Lösung des Wirkstoffes in einem Aceton/Wasser-Gemisch (1:1) getaucht. Die so behandelten Eiablagen werden dann aus diesem Gemisch herausgenommen und bei 28°C und 60 % relativer Luftfeuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen gemäss den Beispielen 1.1.3. und 1.2. zeigen gute Wirkung im obigen Test.

3.7 Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss den Beispielen 1.1.3. und 1.2. zeigen gute Wirkung in obigem Test.

3.8 Reproduktions-Beeinflussung von Anthonomus grandis

Adulte Anthonomus grandis, die nach dem Schlupf nicht älter als 24 Stunden sind, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht.

Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen

Verbindungen gemäss den Beispielen 1.1.3. und 1.2. zeigen eine gute reproduktionsreduzierende Wirkung im obigen Test.

3.9 Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss den Beispielen 1.1.3. und 1.2. zeigen gute Wirkung in diesem Test.

3.10 Wirkung gegen Epilachna varivestis

Etwa 15-20 cm hohe Phaseolus vulgaris-Pflanzen (Buschbohnen) werden mit wässrigen, den zu pröfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvelen Stadium angesetzt. Ueber die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die Mortalität in Prozenten bestimmt. Zur Auswertung hinsichtlich allfälligem Frassschaden (Antifeeding-Effekt), Entwicklungs- und Häutungsstörungen werden die Versuchstiere während weiterer 3 Tage beobachtet.

Verbindungen gemäss den Beispielen 1.1.3. und 1.2. zeigen gute Wirkung im obigen Test.

3.11 Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28°C und 60 % relativer Luftfeuchtigkeit in der Dunkelheit aufbewahrt. Nach 5 bis 8 Tagen wird die Schlupfrate, d.h. die Anzahl Larven,

die sich aus den behandelten Eiern entwickelt haben, gegenüber unbehandelten Kontrollansätzen bestimmt.

Verbindungen gemäss den Beispielen 1.1.3. und 1.2. zeigen in obigem Test eine 80-100%ige ovizide Wirkung (Mortalität) gegen Heliothis virescens und Spodoptera littoralis.

3.12 Wirkung gegen pflanzenschädigende Akarinen: Tetranychus urticae (OP-sensible) und Tetranychus cinnabarinus (OP-tolerant)

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranchus cinnabarinus (OP-tol.) belegt (die Toleranz bezieht sich auf die Verträglichkeit gegenüber Diazinon).

Die so behandelten infestierten Pflanzen werden mit einer Versuchslösung enthaltend 0,75 bis 400 ppm der jeweils zu prüfenden Verbindung bis zur Tropfnässe besprüht. Nach 24 Stunden und wiederum nach 7 Tagen werden Imagines und Larven (alle beweglichen Stadien) unter dem Binolular auf lebende und tote Individuen ausgewertet. Man verwendet pro Konzentration und pro Testspezies eine Pflanze. Während des Versuchsverlaufs stehen die Pflanzen in Gewächshauskabinen bei 25°C.

Verbindungen gemäss den Beispielen 1.1.3. und 1.2. zeigen in diesem Versuch gute Wirkung gegen Tetranychus urticae, und Tetranychus cinnabarinus.

**Patentansprüche**

1. Verbindungen der Formel I

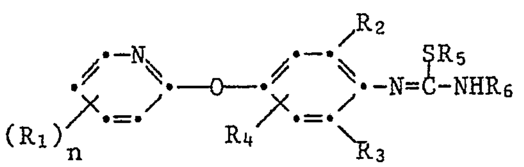

(I)

worin

$R_1$ je für Halogen, $C_1$-$C_3$-Alkyl, ein ein- oder mehrfach halogeniertes $C_1$-$C_3$-Alkyl oder $-COOR_7$,

$R_2$ $R_3$ und $R_7$ je für $C_1$-$C_5$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind,

$R_4$ für Wasserstoff oder Methyl,

$R_5$ für $C_1$-$C_6$-Alkyl,

$R_6$ für $C_1$-$C_{12}$-Alkyl, ein ein- oder mehrfach halogeniertes $C_1$-$C_{12}$-Alkyl, $C_1$-$C_6$-Alkoxy-$C_1$-$C_{12}$-alkyl, $C_3$-$C_8$-Cycloalkyl, ein ein- oder zweifach $C_1$-$C_3$-alkyliertes $C_3$-$C_8$-Cycloalkyl, $C_3$-$C_8$-Cycloalkyl-$C_1$-$C_4$-alkyl oder einen polycyclischen Alkylrest mit 7 bis 10 C-Atomen und

n für eine Zahl von 0 bis 4

stehen, und ihre Salze mit organischen oder anorganischen Säuren.

2. Verbindungen der Formel I gemäss Anspruch 1, worin $R_1$ je für Fluor, Chlor oder ein ein- oder mehrfach halogeniertes $C_1$-$C_3$-Alkyl, $R_2$ und $R_3$ je für $C_1$-$C_3$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind, $R_4$ für Wasserstoff, $R_5$ für $C_1$-$C_3$-Alkyl, $R_6$ für $C_1$-$C_8$-Alkyl, $C_1$-$C_4$-Alkoxy-$C_1$-$C_4$-alkyl, $C_3$-$C_6$-Cycloalkyl, ein ein- oder zweifach $C_1$-$C_3$-alkyliertes $C_3$-$C_6$-Cycloalkyl oder $C_3$-$C_6$-Cycloalkyl-$C_1$-$C_3$-alkyl und n für die Zahl 1 oder 2 stehen.

3. Verbindungen der Formel I gemäss Anspruch 2, worin $R_1$ je für Chlor oder $-CF_2CFCl_2$, $R_2$ und $R_3$ je für Methyl oder Aethyl, mit der Massgabe, dass $R_2$ und $R_3$ nicht gleichzeitig Methyl bedeuten, wenn $R_6$ tert. Butyl und $R_1$ Chlor und/oder Trifluormethyl sind, $R_4$ für Wasserstoff, $R_5$ für Methyl, $R_6$ für $C_1$-$C_5$-Alkyl oder $C_3$-$C_6$-Cycloalkyl und n für 2 stehen.

4. Die Verbindung gemäss Anspruch 3 der Formel

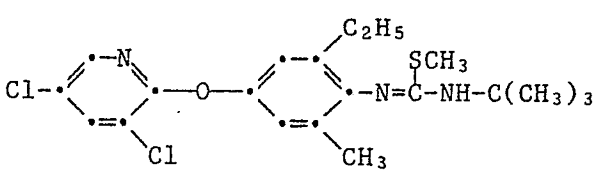

.

5. Die Verbindung gemäss Anspruch 2 der Formel

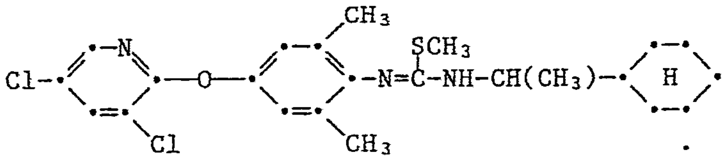

6. Die Verbindung gemäss Anspruch 3 der Formel

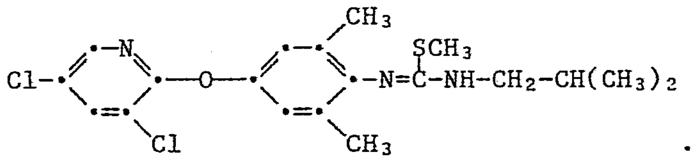

7. Die Verbindung gemäss Anspruch 3 der Formel

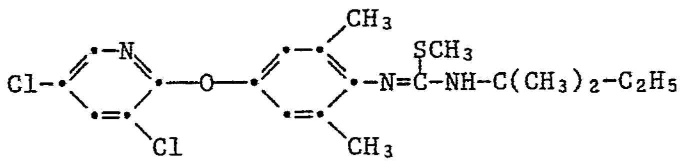

8. Die Verbindung gemäss Anspruch 2 der Formel

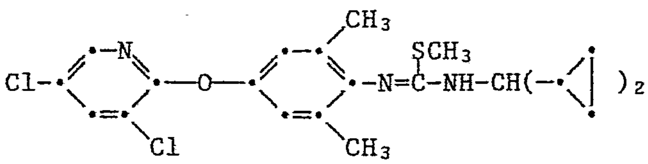

9. Die Verbindung gemäss Anspruch 3 der Formel

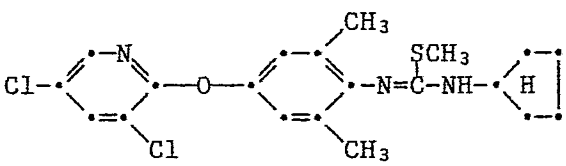

10. Die Verbindung gemäss Anspruch 2 der Formel

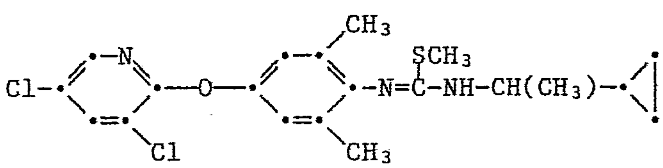

11. Die Verbindung gemäss Anspruch 3 der Formel

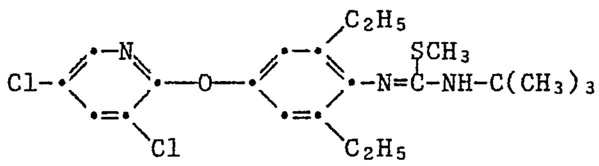

12. Die Verbindung gemäss Anspruch 3 der Formel

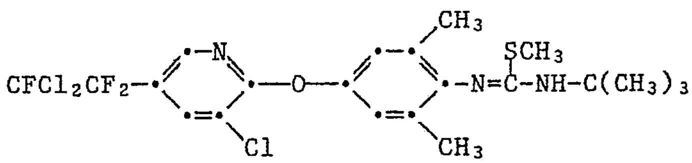

.

13. Die Verbindung gemäss Anspruch 3 der Formel

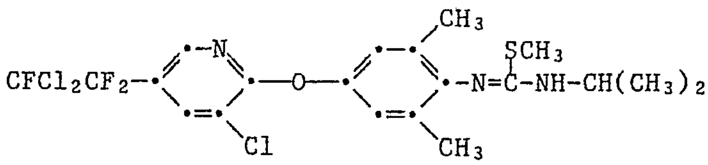

.

14. Die Verbindung gemäss Anspruch 2 der Formel

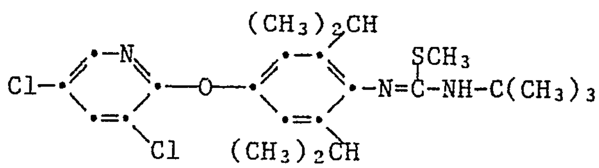

15. Die Verbindung gemäss Anspruch 2 der Formel

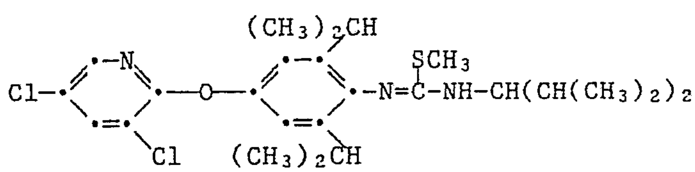

16. Verfahren zur Herstellung einer Verbindung der Formel I

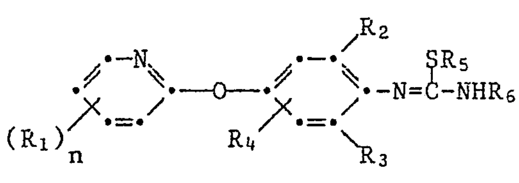

(I)

worin
$R_1$ je für Halogen, C-$C_3$-Alkyl, ein ein- oder mehrfach halogeniertes $C_1$-$C_3$-Alkyl oder -$COOR_7$,
$R_2$ $R_3$ und $R_7$ je für $C_1$-$C_5$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind,
$R_4$ für Wasserstoff oder Methyl,
$R_5$ für $C_1$-$C_6$-Alkyl,
$R_6$ für $C_1$-$C_{12}$-Alkyl, ein ein- oder mehrfach halogeniertes $C_1$-$C_{12}$-Alkyl, $C_1$-$C_6$-Alkoxy-$C_1$-$C_{12}$-alkyl, $C_3$-$C_8$-Cycloalkyl, ein ein- oder zweifach $C_1$-$C_3$-alkyliertes $C_3$-$C_8$-Cycloalkyl, $C_3$-$C_8$-Cycloalkyl-$C_1$-$C_4$-al-

kyl oder einen polycyclischen Alkylrest mit 7 bis 10 C-Atomen und
n für eine Zahl von 0 bis 4
stehen, dadurch gekennzeichnet, dass man einen Pyridyloxyphenylthioharnstoff der Formel II

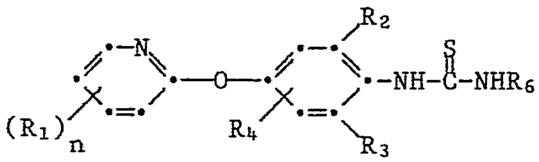
(II),

mit einem Alkylierungsmittel der Formel III
$R_5$-X (III)
umsetzt, worin $R_1$, $R_2$, $R_3$, $R_4$, $R_5$, $R_6$ und n die angegebene Bedeutung haben und X für eine Abgangsgruppe steht.

17. Schädlingsbekämpfungsmittel, welches als aktive Komponente mindestens eine Verbindung der Formel I

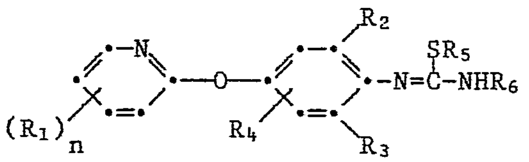
(I),

oder eines ihrer Salze mit organischen oder anorganischen Säuren oder eine Verbindung der Formel II

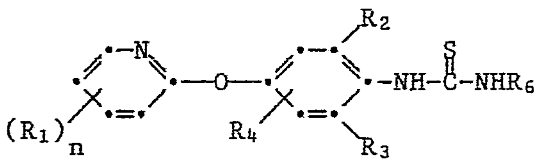
(II),

enthält, worin
$R_1$ je für Halogen, $C_1$-$C_3$-Alkyl, ein ein- oder mehrfach halogeniertes $C_1$-$C_3$-Alkyl oder -$COOR_7$,
$R_2$ $R_3$ und $R_7$ je für $C_1$-$C_5$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind,
$R_4$ für Wasserstoff oder Methyl,
$R_5$ für $C_1$-$C_6$-Alkyl,
$R_6$ für $C_1$-$C_{12}$-Alkyl, ein ein- oder mehrfach halogeniertes $C_1$-$C_{12}$-Alkyl, $C_1$-$C_6$-Alkoxy-$C_1$-$C_{12}$-alkyl, $C_3$-$C_8$-Cycloalkyl, ein ein-oder zweifach $C_1$-$C_3$-alkyliertes $C_3$-$C_8$-Cycloalkyl, $C_3$-$C_8$-Cycloalkyl-$C_1$-$C_4$-alkyl oder einen polycyclischen Alkylrest mit 7 bis 10 C-Atomen und
n für eine Zahl von 0 bis 4
stehen, zusammen mit geeigneten Trägern und/oder Zuschlagstoffen

18. Schädlingsbekämpfungsmittel gemäss Anspruch 17, welches als aktive Komponente mindestens eine Verbindung der Formel I oder II enthält, worin $R_1$ je für Fluor, Chlor oder ein ein- oder mehrfach halogeniertes $C_1$-$C_3$-Alkyl, $R_2$ und $R_3$ je für $C_1$-$C_3$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind, $R_4$ für Wasserstoff, $R_5$ für $C_1$-$C_3$-Alkyl, $R_6$ für $C_1$-$C_8$-Alkyl, $C_1$-$C_4$-Alkoxy-$C_1$-$C_4$-alkyl, $C_3$-$C_6$-Cycloalkyl, ein ein- oder zweifach $C_1$-$C_3$-alkyliertes $C_3$-$C_6$-Cycloalkyl oder $C_3$-$C_6$-Cycloalkyl-$C_1$-$C_3$-alkyl und n für die Zahl 1 oder 2 stehen.

19. Schädlingsbekämpfungsmittel gemäss Anspruch 18, welches als aktive Komponente mindestens eine Verbindung der Formel I oder II enthält, worin $R_1$ je für Chlor oder -$CF_2CFCl_2$, $R_2$ und $R_3$ je für Methyl oder Aethyl, mit der Massgabe, dass $R_2$ und $R_3$ nicht gleichzeitig Methyl bedeuten, wenn $R_6$ tert. Butyl und $R_1$ Chlor und/oder Trifluormethyl sind, $R_4$ für Wasserstoff, $R_5$ für Methyl, $R_6$ für $C_1$-$C_5$-Alkyl oder $C_3$-$C_6$-Cycloalkyl und n für 2 stehen.

20. Verwendung einer Verbindung der Formel I

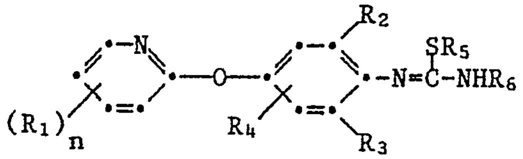

(I)

oder eines ihrer Salze mit organischen oder anorganischen Säuren oder einer Verbindung der Formel II

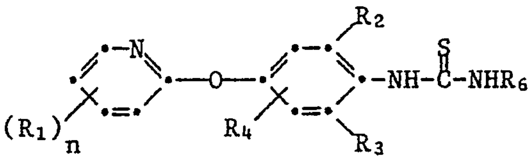

(II)

worin
$R_1$ je für Halogen, $C_1$-$C_3$-Alkyl, ein ein- oder mehrfach halogeniertes $C_1$-$C_3$-Alkyl oder -$COOR_7$,
$R_2$ $R_3$ und $R_7$ je für $C_1$-$C_5$-Alkyl, mit der Massgabe, dass $R_2$ und $R_3$ nicht gleichzeitig Methyl bedeuten, wenn $R_6$ tert.Butyl und $R_1$ Chlor und/oder Trifluormethyl sind,
$R_4$ für Wasserstoff oder Methyl,
$R_5$ für $C_1$-$C_6$-Alkyl,
$R_6$ für $C_1$-$C_{12}$-Alkyl, ein ein- oder mehrfach halogeniertes $C_1$-$C_{12}$-Alkyl, $C_1$-$C_6$-Alkoxy-$C_1$-$C_{12}$-alkyl, $C_3$-$C_8$-Cycloalkyl, ein ein- oder zweifach $C_1$-$C_3$-alkyliertes $C_3$-$C_8$-Cycloalkyl, $C_3$-$C_8$-Cycloalkyl-$C_1$-$C_4$-alkyl oder einen polycyclischen Alkylrest mit 7 bis 10 C-Atomen und
n für eine Zahl von 0 bis 4
stehen zur Bekämpfung von Schädlingen an Tieren und Pflanzen.

21. Verwendung gemäss Anspruch 20 einer Verbindung der Formel I oder II zur Bekämpfung von Insekten und Arachniden.